# EUROPEAN PATENT SPECIFICATION

(11) **EP 2 218 789 B1**
(45) Date of publication and mention of the grant of the patent: **29.02.2012**
(21) Application number: 10151138.4
(22) Date of filing: 11.03.2005
(51) Int. Cl.: C12P 13/08, C12P 13/12, C12P 13/04, C12N 1/20, C07K 14/34

(54) **Process for the production of L-methionine using coryneform bacteria**
Verfahren zur Herstellung von L-Methionin unter Verwendung coryneformer Bakterien
Procédé de production de L-méthionine utilisant des bactéries coryneformes

(30) Priority: 18.03.2004 DE 102004013503
(43) Date of publication of application: 18.08.2010
(62) Divisional of application: 05716004.6
(73) Proprietor: Evonik Degussa GmbH, 45128 Essen (DE)
(72) Inventor: Bathe, Brigitte, 33154, Salzkotten (DE); Schischka, Natalie, 33649, Bielefeld (DE); Pfefferle, Walter, 35428, Langgöns (DE)

(56) References cited:
- EP-A2- 1 108 790
- WO-A-02/097096
- TURNER J S ET AL: "ZN2+-SENSING BY THE CYANOBACTERIAL METALLOTHIONEIN REPRESSOR SMTB: DIFFERENT MOTIFS MEDIATE METAL-INDUCED PROTEIN-DNA DISSOCIATON" NUCLEIC ACIDS RESEARCH, IRL PRESS LTD., OXFORD, GB, vol. 24, no. 19, 1996, pages 3714-3721, XP001119095 ISSN: 0305-1048
- LEE H-S ET AL: "Methionine biosynthesis and its regulation in Corynebacterium glutamicum: Parallel pathways of transsulfuration and direct sulfhydrylation" APPLIED MICROBIOLOGY AND BIOTECHNOLOGY, SPRINGER VERLAG, BERLIN, DE LNKD- DOI:10.1007/S00253-003-1306-7, vol. 62, no. 5-6, 1 October 2003 (2003-10-01), pages 459-467, XP002355864 ISSN: 0175-7598
- RÜCKERT C ET AL: "Genome-wide analysis of the L-methionine biosynthetic pathway in Corynebacterium glutamicum by targeted gene deletion and homologous complementation" JOURNAL OF BIOTECHNOLOGY, ELSEVIER SCIENCE PUBLISHERS, AMSTERDAM, NL LNKD- DOI:10.1016/S0168-1656(03)00158-5, vol. 104, no. 1-3, 4 September 2003 (2003-09-04), pages 213-228, XP002329882 ISSN: 0168-1656
- KALINOWSKI J ET AL: "THE COMPLETE CORYNEBACTERIUM GLUTAMICUM ATCC 13032 GENONE SEQUENCE AND ITS IMPACT ON THE PRODUCTION OF L-ASPARTATE-DERIVED AMINO ACIDS AND VITAMINS" JOURNAL OF BIOTECHNOLOGY, ELSEVIER SCIENCE PUBLISHERS, AMSTERDAM, NL LNKD- DOI:10.1016/S0168-1656(03)00154-8, vol. 104, no. 1-03, 4 September 2003 (2003-09-04), pages 5-25, XP001184752 ISSN: 0168-1656

## Description

The invention provides a process for the production of L-methionine, using coryneform bacteria in which the gene arsR has been attenuated, especially excluded. The mentioned gene codes for a protein from the group of the transcription regulators.

### Prior art

Chemical compounds, which means especially L-amino acids, vitamins, nucleosides and nucleotides and D-amino acids, are used in human medicine, in the pharmaceutical industry, in cosmetics, in the foodstuffs industry and in animal feeds.

Many of these compounds are produced by fermentation of strains of coryneform bacteria, especially Corynebacterium glutamicum. Because of their great importance, work is constantly being carried out to improve the production processes. Improvements to the processes may be concerned with measures relating to the fermentation, such as, for example, stirring and oxygen supply, or the composition of the nutrient media, such as, for example, the sugar concentration during the fermentation, or working up to the product form by, for example, ion-exchange chromatography, or the intrinsic performance properties of the microorganism itself.

In order to improve the performance properties of such microorganisms, methods of mutagenesis, selection and mutant selection are used. These methods yield strains which are resistant to antimetabolites, such as, for example, the lysine analogue S-(2-aminomethyl)-cysteine or the methionine analogues α-methyl-methionine, ethionine, norleucine, N-acetylnorleucine, S-trifluoromethylhomocysteine, 2-amino-5-heprenoitic acid, seleno-methionine, methionine sulfoximine, methoxine, 1-aminocyclopentanecarboxylic acid, or which are auxotrophic for metabolites that are important in terms of regulation, and which produce L-amino acids.

For some years, methods of recombinant DNA technology have also been used to improve the strain of L-amino-acid-producing strains of Corynebacterium glutamicum by amplifying individual amino acid biosynthesis genes and studying the effect on L-amino acid production.

WO02/097096 discloses a process preferably for producing L-methionine by fermentation of coryneform bacteria in which the transcription regulator gene metD is attenuated and WO 02/18596 discloses a process preferably for producing L-lysine, in which the transcription regulator gene citB is attenuated.

### Object of the invention

The inventors have set themselves the object of providing novel bases for improved processes for the production of L-methionine, by fermentation using coryneform bacteria.

### Description of the invention

Any mention of L-amino acids or amino acids hereinbelow means the proteinogenic amino acid L-methionine including its salts.

Proteinogenic amino acids are understood as being the amino acids that occur in natural proteins, that is to say in proteins of microorganisms, plants, animals and humans. They act as structural units for proteins, in which they are linked together via peptide bonds.

Any mention of L-lysine or lysine hereinbelow means not only the bases but also the salts, such as, for example, lysine monohydrochloride or lysine sulfate.

Any mention of L-methionine or methionine hereinbelow also means the salts, such as, for example, methionine hydrochloride or methionine sulfate.

The invention provides a process for the production of L-methionine using coryneform bacteria which, in particular, already produce L-amino acids and in which the gene arsR having the sequence of SEQ ID NO: 500 of EP1108790 (= 
ATGACCTCCGTTATCCCATCCAGCGCAGACAGTGCCACAGAGCAGCGCTTCGCGCCGCTAAAATCCGTAC
CCAGCAGGATCACGGACATCGCCGAAGAGGCCAGCGATTTCTACAAGGCACTCGGTGACACCACCCGCCT
AGAAATCTTGTACCTGATTTTCACTACAAAAAGCTCGCGGGTGAGCGCGAATGCCCTGGCACACACACTC
AATATTTCCGCCCCCACGGTGACCCACCATATGAAAAAGCTCATCGCAGCTAATTTGGTCAACCGTGAAC
AATGCGGAAAGTGGGCGTATTTCAGCATTAATTCCGCTCATATTGATACGGTTGCTGAAATTTTCGCCCA
CGCGTCC)
 which codes for the transcription regulator ArsR is attenuated, especially excluded or expressed at a low level.

This invention further provides a process for the production of L-methionine, in which the following steps are carried out:
a) fermentation of the L-methionine-producing coryneform bacteria, in which the gene arsR is attenuated, especially excluded or expressed at a low level,
b) concentration of the L-amino acid in the medium or in the cells of the bacteria, and
c) isolation of the desired L-amino acid, portions (> 0 to 100 %) or the totality of constituents of the fermentation liquor and/or of the biomass optionally remaining in the end product.

The coryneform bacteria used preferably already produce L-amino acid L-methionine, before the attenuation of the gene

It has been found that coryneform bacteria produce L-methionine, in an improved manner after attenuation of the gene

The gene product of the gene, arsR that is to be attenuated belongs to the group of the transcription regulators.

Transcription regulators are those proteins that are able to bind to DNA by means of a specific protein structure, called the helix-turn-helix motif, and can thus either enhance or attenuate the transcription of other genes. The attenuation of the mentioned transcription regulator improves the production of L-methionine in the corresponding coryneform bacteria.

The nucleotide sequences of the mentioned genes of Corynebacterium glutamicum form part of the prior art and are to be found in various patent applications and also from the data bank of the National Center for Biotechnology Information (NCBI) of the National Library of Medicine (Bethesda, MD, USA).

| smtB gene: | |
|---|---|
| Name: | transcription repressor SmtB |
| Function: | metal-dependent repressor of the expression of the smtA gene coding for a metallothionein, regulation of the cell response to various heavy metals, ArsR family |
| References: | sequences no. 3056 and no. 7068 from EP1108790; sequence no. 559 from WO 01/00805 |
| Accession No.: | AX123140, AX127152 and AX066977 |
| | |

| cgl1 gene: | |
|---|---|
| Name: | transcription regulator Cgl1 |
| Function: | transcription regulator of the GntR family |
| References: | sequences no. 3251 and no. 7069 from EP1108790 |
| Accession No.: | AX123335 and AX127153 |
| | |

| hspR gene: | |
|---|---|
| Name: | heat-shock regulator protein HspR |
| Function: | binds in the promotor region upstream of the gene dnaK coding for a chaperone |
| References: | sequences no. 3073 and no. 7068 from EP1108790 |
| Accession No.: | AX123157 and AX127152 |
| | |

| cgl2 gene: | |
|---|---|
| Name: | transcription regulator Cgl2 |
| Function: | transcription regulator of the TetR family |
| References: | sequences no. 1700 and no. 7064 from EP1108790 |
| Accession No.: | AX121784 and AX127148 |

| cebR gene: | |
|---|---|
| Name: | transcription regulator CebR |
| Function: | regulation of the uptake of cellobiose and cellotriose |
| References: | sequences no. 2889 and no. 7068 from EP1108790 |
| Accession No.: | AX122973 and AX127152 |
| | |

| cgl3 gene: | |
|---|---|
| Name: | transcription regulator Cgl3 |
| Function: | transcription regulator of the TetR family |
| References: | sequences no. 3379 and no. 7069 from EP1108790 |
| Accession No.: | AX123463 and AX127153 |
| | |

| gatR gene: | |
|---|---|
| Name: | transcription regulator GatR |
| Function: | repressor of the Gat operon for uptake and utilisation of galacticol |
| References: | sequences no. 129 and no. 1 from EP1108790, sequence no. 195 from WO 01/00844 |
| Accession No.: | AX120213, AX120085 and AX065069 |
| | |

| glcR gene: | |
|---|---|
| Name: | transcription regulator GlcR |
| Function: | transcription regulator of the DeoR family |
| References: | sequences no. 123 and no. 1 from EP1108790 |
| Accession No.: | AX120207 and AX120085 |
| | |

| tcmR gene: | |
|---|---|
| Name: | transcription repressor TcmR |
| Function: | regulation of tetracenomycin resistance and export, TetR family |
| References: | sequences no. 424 and no. 7060 from EP1108790; the bases 506 to 606 of the coding region are in each case contained in sequences 247 and 249 from WO 01/00804 |
| Accession No.: | AX120508 and AX127144; AX066343 and AX066345 |

| smtB2 gene: | |
|---|---|
| Name: | transcription repressor SmtB2 |
| Function: | metal-dependent repressor of the expression of the smtA gene coding for a metallothionein, regulation of the cell response to various heavy metals, ArsR family |
| References: | sequences no. 292 and no. 1 from EP1108790, sequence no. 119 from WO 01/00804 |
| Accession No.: | AX120376, AX120085 and AX066215 |
| | |

| dtxR gene: | |
|---|---|
| Name: | diphtheria toxin repressor |
| Function: | iron-binding repressor of diphtheria toxin gene expression |
| References: | Oguiza *et al*., Journal of Bacteriology 177 (2), 465-467 (1995) |
| Accession No.: | L35906 |
| | |

| degA gene: | |
|---|---|
| Name: | degradation regulator DegA |
| Function: | involved in controlling the inactivation and degradation of enzymes, LacI family |
| References: | sequences no. 2240 and no. 7966 from EP1108790, sequence no. 337 from WO 01/00844 |
| Accession No.: | AX122324, AX127150 and AX065211 |
| | |

| galR gene: | |
|---|---|
| Name: | galactose operon repressor |
| Function: | galactose-inducible repressor of the galactose operon |
| References: | sequences no. 2309 and no. 7066 from EP1108790, sequence no. 383 from WO 01/00844 |
| Accession No.: | AX122393, AX127150 and AX065257 |

| tipA2 gene: | |
|---|---|
| Name: | transcription regulator TipA2 |
| Function: | transcription regluator of the MerR family |
| References: | sequences no. 2349 and no. 7066 from EP1108790 |
| Accession No.: | AX122433 and AX127150 |
| | |

| malI gene: | |
|---|---|
| Name: | maltose regulon repressor |
| Function: | maltose-inducible, LacI family |
| References: | from EP1108790: nucleotides 149 to 601 in sequence no. 2724, nucleotides 1 to 346 in sequence no. 2725, nucleotides 539 to 1080 in no. 2727 and the whole of the coding region in no. 7067 |
| Accession No.: | AX122808, AX122809, AX122811 and AX127151 |
| | |

| cgl4 gene: | |
|---|---|
| Name: | transcription regulator Cgl4 |
| References: | sequence no. 972, no. 7061 and no. 7062 from EP1108790, nucleotides 205 to 309 in sequence no. 617 from WO 01/00805 |
| Accession No.: | AX121056, AX127145, AX127146 and AX067035 |
| | |

| arsR gene: | |
|---|---|
| Name: | transcription regulator ArsR |
| Function: | regulation and expression of the arsenic resistance operon |
| References: | sequence no. 500 and no. 7060 from EP1108790 |
| Accession No.: | AX120584 and AX127144 |
| | |

| merR gene: | |
|---|---|
| Name: | mercury resistance operon regulator |
| Function: | mediates mercury-dependent induction of the mercury resistance operon, MerR family |
| References: | sequence no. 1009 and no. 7062 from EP1108790 |
| Accession No.: | AX127146 and AX121093 |

| hrcA gene: | |
|---|---|
| Name: | transcription repressor HrcA |
| Function: | heat-inducible transcription repressor of heat-shock proteins, HrcA family |
| References: | sequence no. 2513, no. 7066 and no. 7067 from EP1108790, |
| Accession No.: | AX127150, AX127150 and AX127151 |
| | |

| glpR2 gene: | |
|---|---|
| Name: | glycerol-3-phosphate regulon repressor |
| Function: | regulation of the genes of glycerol metabolism, DeoR family |
| References: | sequence no. 2123 and no. 7065 from EP1108790, nucleotides 881 to 981 in sequence no. 57 from WO 01/00845 |
| Accession No.: | AX122207, AX127149 and AX064931 |
| | |

| lexA: | |
|---|---|
| Name: | SOS regulon repressor LexA (EC No. 3.4.21.88) |
| Function: | mediates SOS-induced expression of DNA repair proteins, peptidase family S24 |
| References: | sequence no. 2119 and no. 7065 from EP1108790 |
| Accession No.: | AX122203 and AX127149 |
| | |

| ccpA3: | |
|---|---|
| Name: | catabolite control protein CcpA3 |
| Function: | involved in the repression of carbohydrate-degrading genes and in the positive regulation of genes for the excretion of excess carbon, LacI family |
| References: | sequence no. 200 and no. 1 from EP1108790 |
| Accession No.: | AX120284 and AX120085 |

| degA2: | |
|---|---|
| Name: | degradation regulator DegA2 |
| Function: | involved in controlling the inactivation and degradation of enzymes, LacI family |
| References: | sequence no. 191 and no. 1 from EP1108790 |
| Accession No.: | AX120275 and AX120085 |

The sequences described in the indicated references and coding for the gene arsR can be used in accordance with the invention. It is also possible to use alleles of the mentioned gene, which result from the degeneracy of the genetic code or by function-neutral sense mutations.

Preferred embodiments are to be found in the claims.

The term "attenuation" or "attenuate" in this context describes reducing or excluding the intracellular activity of one or more enzymes or proteins in a microorganism that are coded for by the corresponding DNA, by, for example, using a weak promoter or using a gene or allele that codes for a corresponding enzyme having a low level of activity, or by rendering the corresponding gene or enzyme or protein inactive, and optionally combining these measures.

By the measures of the attenuation, the activity or concentration of the corresponding protein is generally lowered to 0 to 75 %, 0 to 50 %, 0 to 25 %, 0 to 10 % or 0 to 5 % of the activity or concentration of the wild-type protein, or of the activity or concentration of the protein in the starting microorganism.

The reduction in the protein concentration can be demonstrated by 1- and 2-dimensional protein gel separation and subsequent optical identification of the protein concentration using corresponding evaluation software in the gel. A common method for preparing the protein gels in the case of coryneform bacteria and for identifying the proteins is the procedure described by Hermann et al. (Electrophoresis, 22:1712-23 (2001)). The protein concentration can also be analysed by Western blot hybridisation using a specific antibody for the protein to be demonstrated (Sambrook et al., Molecular Cloning: A Laboratory Manual, 2nd Ed., Cold Spring Harbor Laboratory Press, Cold Spring Harbor, N.Y., 1989) and subsequent optical evaluation using appropriate software for concentration determination (Lohaus and Meyer (1998) Biospektrum 5:32-39; Lottspeich, Angewandte Chemie 111: 2630-2647 (1999)). The activity of DNA-binding proteins can be measured by means of DNA band-shift assays (also referred to as gel retardation), as described, for example, in the textbook "Bioanalytik" (Lottspeich/Zorbas, Spektrum Akademischer Verlag GmbH, Heidelberg, Germany, 1998) and used by Wilson et al. (J. Bacteriol. 183:2151-2155 (2001)). The effect of DNA-binding proteins on the expression of other genes can be demonstrated by various well described methods of the reporter gene assay (Sambrook et al., Molecular Cloning: A Laboratory Manual, 2nd Ed., Cold Spring Harbor Laboratory Press, Cold Spring Harbor, N.Y., 1989).

The microorganisms provided by the present invention are able to produce amino acids from glucose, sucrose, lactose, fructose, maltose, molasses, starch, cellulose or from glycerol and ethanol. They are representatives of coryneform bacteria, especially of the genus Corynebacterium. In the case of the genus Corynebacterium, particular mention is to be made of the species Corynebacterium glutamicum, which is known to those skilled in the art for its ability to produce L-amino acids.

Suitable strains of the genus Corynebacterium, especially of the species Corynebacterium glutamicum, are in particular the known wild-type strains
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 and
Brevibacterium divaricatum ATCC14020
and L-amino-acid-producing mutants and strains produced therefrom,
such as, for example, the L-methionine-producing strain Corynebacterium glutamicum ATCC21608.

Strains designated "ATCC" can be obtained from the American Type Culture Collection (Manassas, VA, USA). Strains designated "FERM" can be obtained from the National Institute of Advanced Industrial Science and Technology (AIST Tsukuba Central 6, 1-1-1 Higashi, Tsukuba Ibaraki, Japan). The mentioned strain of Corynebacterium thermoaminogenes (FERM BP-1539) is described in US-A-5,250,434.

In order to achieve an attenuation, either the expression of the genes or the regulatory properties of the gene products can be reduced or excluded. The two measures are optionally combined.

Gene expression can be diminished by carrying out the culturing in a suitable manner or by genetic alteration (mutation) of the signal structures of gene expression. Signal structures of gene expression are, for example, repressor genes, activator genes, operators, promoters, attenuators, ribosome-binding sites, the start codon and terminators. The person skilled in the art will find information thereon, for example, in patent application WO 96/15246, in Boyd and Murphy (Journal of Bacteriology 170: 5949-5952 (1988)), in Voskuil and Chambliss (Nucleic Acids Research 26: 3584-3590 (1998), in Pátek et al. (Microbiology 142: 1297-309 (1996) and Journal of Biotechnology 104: 311-323 (2003)), and in known textbooks of genetics and molecular biology, such as, for example, the textbook of Knippers ("Molekulare Genetik", 6th edition, Georg Thieme Verlag, Stuttgart, Germany, 1995) or that of Winnacker ("Gene und Klone", VCH Verlagsgesellschaft, Weinheim, Germany, 1990).

An example of the targeted regulation of gene expression is the cloning of the gene that is to be attenuated, under the control of a promoter inducible by the addition of metered amounts of IPTG (isopropyl-β-D-thiogalactopyranoside), such as, for example, the trc promoter or the tac promoter. Suitable for this purpose are vectors such as, for example, the Escherichia coli expression vector pXK99E (WO0226787; deposited in accordance with the Budapest Treaty on 31 July 2001 in DH5alpha/pXK99E as DSM14440 at the Deutsche Sammlung für Mikroorganismen und Zellkulturen (DSMZ, Braunschweig, Germany)) or pVWEx2 (Wendisch, Ph. D. thesis, Berichte des Forschungszentrums Jülich, Jül-3397, ISSN 0994-2952, Jülich, Germany (1997)), which permit IPTG-dependent expression of the cloned gene in Corynebacterium glutamicum.

This method has been used, for example, in patent specification WO0226787 for the regulated expression of the deaD gene by integration of the vector pXK99EdeaD into the genome of Corynebacterium glutamicum, and by Simic et al. (Applied and Environmental Microbiology 68: 3321-3327 (2002)) for the regulated expression of the glyA gene by integration of the vector pK18mobglyA' in Corynebacterium glutamicum.

A further method of specifically reducing gene expression is the antisense technique, in which short oligodeoxynucleotides or vectors are introduced into the target cells for the synthesis of longer antisense RNA. The antisense RNA is there able to bind to complementary sections of specific mRNAs and reduce their stability or block the translatability. The person skilled in the art will find an example thereof in Srivastava et al. (Applied Environmental Microbiology 2000 Oct; 66 (10): 4366-4371).

Mutations that lead to a change in or diminution of the catalytic properties of enzyme proteins are known from the prior art; examples which may be mentioned include the works of Qiu and Goodman (Journal of Biological Chemistry 272: 8611-8617 (1997)), Sugimoto et al. (Bioscience Biotechnology and Biochemistry 61: 1760-1762 (1997)) and Möckel ("Die Threonindehydratase aus Corynebacterium glutamicum: Aufhebung der allosterischen Regulation und Struktur des Enzyms", Berichte des Forschungszentrums Jülich, Jül-2906, ISSN09442952, Jülich, Germany, 1994). Summaries are to be found in known textbooks of genetics and molecular biology, such as, for example, that of Hagemann ("Allgemeine Genetik", Gustav Fischer Verlag, Stuttgart, 1986).

As mutations there come into consideration transitions, transversions, insertions and deletions. Depending on the effect of the amino acid substitution on the enzyme activity, the terms missense mutations or nonsense mutations are used. Insertions or deletions of at least one base pair in a gene lead to frame shift mutations, as a result of which incorrect amino acids are incorporated or the translation breaks off prematurely. Deletions of several codons typically lead to complete loss of enzyme activity. Instructions for the production of such mutations form part of the prior art and can be found in known textbooks of genetics and molecular biology, such as, for example, the textbook of Knippers ("Molekulare Genetik", 6th edition, Georg Thieme Verlag, Stuttgart, Germany, 1995), that of Winnacker ("Gene und Klone", VCH Verlagsgesellschaft, Weinheim, Germany, 1990) or that of Hagemann ("Allgemeine Genetik", Gustav Fischer Verlag, Stuttgart, 1986).

A common method of mutating genes of C. glutamicum is the method of gene disruption and gene replacement described by Schwarzer and Pühler (Bio/Technology 9, 84-87 (1991)).

In the method of gene disruption, for example a central portion of the coding region of the gene in question is cloned into a plasmid vector which is capable of replication in a host (typically E. coli) but not in C. glutamicum. Suitable vectors are, for example, pSUP301 (Simon et al., Bio/Technology 1, 784-791 (1983)), pK18mob, pK19mob, pK18mobsacB or pK19mobsacB (Schäfer et al., Gene 145, 69-73 (1994)), pGEM-T (Promega Corporation, Madison, WI, USA), pCR2.1-TOPO (Shuman (1994), Journal of Biological Chemistry 269:32678-84; US Patent 5,487,993), pCR®Blunt (Invitrogen, Groningen, Netherlands; Bernard et al., Journal of Molecular Biology, 234: 534-541 (1993)) or pEM1 (Schrumpf et al., 1991, Journal of Bacteriology 173: 4510-4516). The plasmid vector containing the central portion of the coding region of the gene is then transferred to the desired strain of C. glutamicum by conjugation or transformation. The method of conjugation is described, for example, in Schäfer et al. (Journal of Bacteriology 172: 1663-1666 (1990) and Applied and Environmental Microbiology 60, 756-759 (1994)). Methods of transformation are described, for example, in Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a cross-over occurrence, the coding region of the gene in question is disrupted by the vector sequence, and two incomplete alleles lacking the 3'- and the 5'-end, respectively, are obtained. This method has been used, for example, by Fitzpatrick et al. (Applied Microbiology and Biotechnology 42, 575-580 (1994)) to exclude the recA gene of C. glutamicum.

In the gene replacement method, a mutation, such as, for example, a deletion, insertion or base substitution, is produced *in vitro* in the gene in question. The allele that is produced is in turn cloned into a vector that is not replicative for C. glutamicum, and the latter is then transferred to the desired host of C. glutamicum by transformation or conjugation. After homologous recombination by means of a first cross-over occurrence effecting integration and by means of a suitable second cross-over occurrence effecting an excision in the target gene or in the target sequence, incorporation of the mutation or of the allele is achieved. This method is described in Scharzer and Pühler (Bio/Technology 9: 84-87 (1991) and has been used, for example, by Peters-Wendisch et al. (Microbiology 144, 915-927 (1998)) to exclude the pyc gene of C. glutamicum by means of a deletion or by Wehmeier et al. (Microbiology 144: 1853-1862 (1988)) to insert a deletion into the rel gene of C. glutamicum.

An overview of various methods of genetic engineering in the case of C. glutamicum is given by Kirchner and Tauch (Journal of Biotechnology 104: 287-299 (2003).

It is possible in this manner to incorporate a deletion, insertion or base substitution into the gene arsR.

It may further be advantageous for the production of L-methionine, in addition to attenuating the gene arSR either to enhance, especially overexpress, or to attenuate, especially exclude or reduce the expression of, one or more enzymes of the biosynthesis pathway in question, of glycolysis, of the anaplerotic pathway, of the citric acid cycle, of the pentose phosphate cycle, of amino acid export, and optionally regulatory proteins.

The term "enhancement" or "enhance" in this context describes increasing the intracellular activity or concentration of one or more enzymes or proteins in a microorganism that are coded for by the corresponding DNA, by, for example, increasing the copy number of the gene or genes, using a strong promoter or a gene or allele that codes for a corresponding enzyme or protein having a high level of activity, and optionally combining these measures.

By the measures of the enhancement, especially overexpression, the activity or concentration of the corresponding protein is generally increased by at least 10 %, 25 %, 50 %, 75 %, 100 %, 150 %, 200 %, 300 %, 400 % or 500 %, by a maximum of 1000 % or 2000 %, relative to that of the wild-type protein or to the activity or concentration of the protein in the starting microorganism.

The use of endogenous genes is generally preferred. The expression "endogenous genes" or "endogenous nucleotide sequences" is understood to mean the genes or nucleotide sequences present in the population of a species.

According to the present description it is thus possible, for example, for the production of L-lysine, in addition to attenuating one or more genes selected from the group smtB, cgl1, hspR, cgl2, cebR, cgl3, gatR, glcR, tcmR, smtB2, dtxR, degA, galR, tipA2, malI, cgl4, arsR, merR, hrcA, glpR2, lexA, ccpA3 and degA2, to enhance, especially overexpress, one or more genes selected from the group of the genes or alleles of lysine production. "Genes or alleles of lysine production" are understood as being all those, preferably endogenous, open reading frames, genes or alleles the enhancement/overexpression of which is able to bring about an improvement in lysine production.

These include *inter alia* the following open reading frames, genes or alleles: accBC, accDA, cstA, cysD, cysE, cysH, cysK, cysN, cysQ, dapA, dapB, dapC, dapD, dapE, dapF, ddh, dps, eno, gap, gap2, gdh, gnd, lysC, lysC^{FBR}, lysE, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwal, zwf and zwf A243T, which are compiled and explained in Table 1.

**Table 1**

| Genes and alleles of lysine production | | | |
|---|---|---|---|
| **Name** | **Name of the enzyme or protein coded for** | **Reference** | **Accession number** |
| accBC | acyl-CoA carboxylase EC 6.3.4.14 | Jäger *et al.* Archives of Microbiology (1996) 166:76-82 | U35023 |
| | | EP1108790; | AX123524 |
| | | W00100805 | AX066441 |
| accDA | acetyl-CoA carboxylase | EP1055725 | |
| | EC 6.4.1.2 | EP1108790 | AX121013 |
| | | WO0100805 | AX066443 |
| cstA | carbon starvation protein A | EP1108790 | AX120811 |
| | | WO0100804 | AX066109 |
| cysD | sulfate adenylyltransferase subunit II EC 2.7.7.4, (sulfate adenylyltransferase small chain) | EP1108790 | AX123177 |
| cysE | serine acetyltransferase | EP1108790 | AX122902 |
| | EC 2.3.1.30 | WO0100843 | AX063961 |
| cysH | 3'-phosphoadenyl sulfate reductase | EP1108790 | AX123178 |
| | EC 1.8.99.4 (3'-phosphoadenosine 5'-phosphosulfate reductase) | WO0100842 | AX066001 |
| cysK | cysteine synthase | EP1108790 | AX122901 |
| | EC 4.2.99.8 | WO0100843 | AX063963 |
| cysN | sulfate adenylyltransferase | EP1108790 | AX123176 |
| | subunit I | | AX127152 |
| | EC 2.7.7.4 | | |
| | (sulfate adenylyltransferase) | | |
| cysQ | transport protein CysQ | EP1108790 | AX127145 |
| | (transporter cysQ) | WO0100805 | AX066423 |
| dapA | dihydrodipicolinate synthase EC 4.2.1.52 | Bonnassie *et al*. Nucleic Acids Research 18:6421 (1990) Pisabarro *et al*., Journal of Bacteriology 175:2743-2749(1993) | X53993 |
| | | | Z21502 |
| | | EP1108790 | |
| | | WO0100805 | |
| | | EP0435132 | |
| | | EP1067192 | AX123560 |
| | | EP1067193 | AX063773 |
| dapB | dihydrodipicolinate reductase | EP1108790 | AX127149 |
| | EC 1.3.1.26 | WO0100843 | AX063753 |
| | | EP1067192 | AX137723 |
| | | EP1067193 | AX137602 |
| | | Pisabarro *et al*., Journal of Bacteriology 175:2743-2749(1993) | X67737 |
| | | | Z21502 |
| | | JP1998215883 | |
| | | JP1997322774 | E16749 |
| | | JP1997070291 | E14520 |
| | | JP1995075578 | E12773 |
| | | | E08900 |
| dapC | N-succinyl-aminoketopimelate | EP1108790 | AX127146 |
| | transaminase | WO0100843 | AX064219 |
| | EC 2.6.1.17 | EP1136559 | |
| | (N-succinyl-diaminopimelate transaminase) | | |
| dapD | tetrahydrodipicolinate succinylase | EP1108790 | AX127146 |
| | EC 2.3.1.117 | WO0100843 | AX063757 |
| | | Wehrmann *et al.* Journal of Bacteriology 180:3159-3165(1998) | AJ004934 |
| dapE | N-succinyl-diaminopimelate | EP1108790 | AX127146 |
| | desuccinylase | WO0100843 | AX063749 |
| | EC 3.5.1.18 | Wehrmann *et al*. Microbiology 140:3349-3356 (1994) | X81379 |
| dapF | diaminopimelate epimerase | EP1108790 | AX127149 |
| | EC 5.1.1.7 | WO0100843 | AX063719 |
| | | EP1085094 | AX137620 |
| ddh | diaminopimelate dehydrogenase | EP1108790 | AX127152 |
| | EC 1.4.1.16 | WO0100843 | AX063759 |
| | | Ishino *et al*., Nucleic Acids Research 15:3917-3917(1987) | Y00151 |
| | | JP1997322774 | |
| | | JP1993284970 | E14511 |
| | | Kim *et al*., Journal of Microbiology and Biotechnology 5:250-256(1995) | E05776 |
| | | | D87976 |
| dps | DNA protection protein (protection during starvation protein) | EP1108790 | AX127153 |
| eno | enolase | EP1108790 | AX127146 |
| | EC 4.2.1.11 | WO0100844 | AX064945 |
| | | EP1090998 Hermann *et al*., Electrophoresis 19:3217-3221 (1998) | AX136862 |
| gap | glyceraldehyde-3-phosphate | EP1108790 | AX127148 |
| | dehydrogenase | WO0100844 | AX064941 |
| | EC 1.2.1.12 | Eikmanns *et al*., Journal of Bacteriology 174:6076-6086(1992) | X59403 |
| gap2 | glyceraldehyde-3-phosphate | EP1108790 | AX127146 |
| | dehydrogenase | WO0100844 | AX064939 |
| | EC 1.2.1.12 | | |
| | (glyceraldehyde-3-phosphate dehydrogenase 2) | | |
| gdh | glutamate dehydrogenase | EP1108790 | AX127150 |
| | EC 1.4.1.4 | WO0100844 | AX063811 |
| | | Boermann *et al*., Molecular Microbiology 6:317-326 (1992) | X59404 |
| | | | X72855 |
| gnd | 6-phosphogluconate dehydrogenase | EP1108790 | AX127147 |
| | EC 1.1.1.44 | | AX121689 |
| | | WO0100844 | AX065125 |
| lysC | aspartate kinase | EP1108790 | AX120365 |
| | EC 2.7.2.4 | WO0100844 | AX063743 |
| | | Kalinowski *et al*., Molecular Microbiology 5:1197-204 (1991) | X57226 |
| lysE | lysine exporter | EP1108790 | AX123539 |
| | (lysine exporter protein) | WO0100843 | AX123539 |
| | | Vrljić *et al*., Molecular Microbiology 22:815-826 (1996) | X96471 |
| msiK | sugar importer | EP1108790 | AX120892 |
| | (multiple sugar import protein) | | |
| opcA | glucose-6-phosphate dehydrogenase (subunit of glucose-6-phosphate dehydrogenase) | WO0104325 | AX076272 |
| oxyR | transcription regulator | EP1108790 | AX122198 |
| | (transcriptional regulator) | | AX127149 |
| ppc^{FBR} | phosphoenolpyruvate carboxylase | EP0723011 | |
| | feedback resistant | WO0100852 | |
| | EC 4.1.1.31 | | |
| ppc | phosphoenolpyruvate carboxylase | EP1108790 | AX127148 |
| | EC 4.1.1.31 | | AX123554 |
| | | O'Reagan *et al*., Gene 77 (2) :237-251 (1989) | M25819 |
| pgk | phosphoglycerate kinase | EP1108790 | AX121838 |
| | EC 2.7.2.3 | | AX127148 |
| | | WO0100844 | AX064943 |
| | | Eikmanns, Journal of Bacteriology 174:6076-6086 (1992) | X59403 |
| pknA | protein kinase A | EP1108790 | AX120131 |
| | | | AX120085 |
| pknB | protein kinase B | EP1108790 | AX120130 |
| | | | AX120085 |
| pknD | protein kinase D | EP1108790 | AX127150 |
| | | | AX122469 |
| | | | AX122468 |
| pknG | protein kinase G | EP1108790 | AX127152 |
| | | | AX123109 |
| ppsA | phosphoenolpyruvate synthase | EP1108790 | AX127144 |
| | EC 2.7.9.2 | | AX120700 |
| | | | AX122469 |
| ptsH | phosphotransferase system protein H | EP1108790 | AX122210 |
| | EC 2.7.1.69 | | AX127149 |
| | (phosphotransferase system component H) | WO0100844 | AX069154 |
| ptsI | phosphotransferase system enzyme I | EP1108790 | AX122206 |
| | EC 2.7.3.9 | | AX127149 |
| ptsM | glucose-specific phosphotransferase system enzyme II EC 2.7.1.69 (glucose-phosphotransferase-system enzyme II) | Lee *et al.,* FEMS Microbiology Letters 119(1-2):137-145 (1994) | L18874 |
| pyc | pyrvate carboxylase | WO9918228 | A97276 |
| | EC 6.4.1.1 | Peters-Wendisch *et al*., Microbiology 144:915-927 (1998) | Y09548 |
| pyc P458S | pyrvate carboxylase EC 6.4.1.1 amino acid substitution P458S | EP1108790 | |
| sigC | sigma factor C | EP1108790 | AX120368 |
| | EC 2.7.7.6 (extracytoplasmic function alternative sigma factor C) | | AX120085 |
| sigD | RNA polymerase sigma factor D | EP1108790 | AX120753 |
| | EC 2.7.7.6 (RNA polymerase sigma factor) | | AX127144 |
| sigE | sigma factor E | EP1108790 | AX127146 |
| | EC 2.7.7.6 (extracytoplasmic function alternative sigma factor E) | | AX121325 |
| sigH | sigma factor H | EP1108790 | AX127145 |
| | EC 2.7.7.6 (sigma factor SigH) | | AX120939 |
| sigM | sigma factor M | EP1108790 | AX123500 |
| | EC 2.7.7.6 (sigma factor SigM) | | AX127153 |
| tal | transaldolase EC 2.2.1.2 | WO0104325 | AX076272 |
| thyA | thymidylate synthase | EP1108790 | AX121026 |
| | EC 2.1.1.45 | | AX127145 |
| tkt | transketolase EC 2.2.1.1 | Ikeda *et al.,* NCBI | AB023377 |
| tpi | triose-phosphate isomerase EC 5.3.1.1 | Eikmanns, Journal of Bacteriology 174:6076-6086 (1992) | X59403 |
| zwal | cell growth factor 1 (growth factor 1) | EP1111062 | AX133781 |
| zwf | glucose-6-phosphate-1-dehydrogenase | EP1108790 | AX127148 |
| | EC 1.1.1.49 | | AX121827 |
| | | WO0104325 | AX076272 |
| zwf A213T | glucose-6-phosphate-1-dehydrogenase EC 1.1.1.49 amino acid substitution A213T | EP1108790 | |

According to the description it may further be advantageous for the production of L-lysine, in addition to attenuating one or more genes selected from the group smtB, cgl1, hspR, cgl2, cebR, cgl3, gatR, glcR, tcmR, smtB2, dtxR, degA, galR, tipA2, malI, cgl4, arsR, merR, hrcA, glpR2, lexA, ccpA3 and degA2, at the same time to attenuate, especially reduce the expression of, one or more genes selected from the group of the genes or alleles that are not essential for growth or for lysine production.

These include *inter alia* the following open reading frames, genes or alleles: aecD, ccpA1, ccpA2, citA, citB, citE, fda, gluA, gluB, gluC, gluD, luxR, luxS, lysR1, lysR2, lysR3, menE, mqo, pck, pgi, poxB and zwa2, which are compiled and explained in Table 2.

**Table 2**

| Genes and alleles that are not essential for lysine production | | | |
|---|---|---|---|
| **Gene name** | **Name of the enzyme or protein coded for** | **Reference** | **Accession number** |
| aecD | beta C-S lyase EC 2.6.1.1 M89931 | Rossol *et al*., Journal of Bacteriology 174(9):2968-77 (1992) | M89931 |
| ccpA1 | catabolite control | WO0100844 | AX065267 |
| | protein (catabolite control protein A1) | EP1108790 WO 02/18419 | AX127147 |
| ccpA2 | catabolite control | WO0100844 | AX065267 |
| | protein (catabolite control protein A2) | EP1108790 | AX121594 |
| citA | sensor kinase CitA | EP1108790 | AX120161 |
| citB | transcription regulator CitB | EP1108790 | AX120163 |
| citE | citrate lyase | WO0100844 | AX065421 |
| | EC 4.1.3.6 | EP1108790 | AX127146 |
| fda | fructose bisphosphate aldolase EC 4.1.2.13 X17313 (fructose 1,6-bisphosphate aldolase) | von der Osten *et al*., Molecular Microbiology 3(11):1625-37 (1989) | X17313 |
| gluA | glutamate transport ATP-binding protein | Kronemeyer *et al.,* Journal of Bacteriology 177(5):1152-8 (1995) | X81191 |
| gluB | glutamate-binding protein | Kronemeyer *et al.,* Journal of Bacteriology 177(5):1152-8 (1995) | X81191 |
| gluC | glutamate transport permease (glutamate transport system permease) | Kronemeyer *et al*., Journal of Bacteriology 177(5):1152-8 (1995) | X81191 |
| gluD | glutamate transport permease (glutamate transport system permease) | Kronemeyer *et al*., Journal of Bacteriology 177(5):1152-8 (1995) | X81191 |
| luxR | transcription regulator | WO0100842 | AX065953 |
| | LuxR | EP1108790 | AX123320 |
| luxS | histidine kinase LuxS | EP1108790 | AX123323 |
| | | | AX127153 |
| lysR1 | transcription regulator | EP1108790 | AX064673 |
| | LysR1 | | AX127144 |
| lysR2 | transcription activator LysR2 (transcription regulator LysR2) | EP1108790 | AX123312 |
| lysR3 | transcription regulator | WO0100842 | AX065957 |
| | LysR3 | EP1108790 | AX127150 |
| menE | O-succinylbenzoic acid | WO0100843 | AX064599 |
| | CoA ligase | EP1108790 | AX064193 |
| | EC 6.2.1.26 (O-succinylbenzoate-CoA ligase) | | AX127144 |
| mqo | malate-quinone-oxidoreductase | Molenaar *et al*., Eur. Journal of Biochemistry 1;254(2):395-403 (1998) | AJ224946 |
| pck | phosphoenolpyruvate | WO0100844 | AJ269506 |
| | carboxykinase | EP-A-1094111 | AX065053 |
| pgi | glucose-6-phosphate | EP1087015 | AX136015 |
| | isomerase EC 5.3.1.9 | EP1108790 WO 01/07626 | AX127146 |
| poxB | pyruvate oxidase | WO0100844 | AX064959 |
| | EC 1.2.3.3 | EP1096013 | AX137665 |
| zwa2 | cell growth factor 2 | EP1106693 | AX113822 |
| | (growth factor 2) | EP1108790 | AX127146 |

It may further be advantageous, for example for the production of L-methionine, if, in addition to attenuating the import of methionine from the surrounding medium, to be achieved, for example, by attenuation of one or more genes selected from the group yaeC, abc and yaeE, one or more genes selected from the group of the genes or alleles of methionine production is/are enhanced, especially overexpressed. "Genes or alleles of methionine production" is understood as meaning all, preferably endogenous, open reading frames, genes or alleles the enhancement/overexpression of which is able to bring about an improvement in methionine production.

These include *inter alia* the following open reading frames, genes or alleles: accBC, accDA, aecD, cstA, cysD, cysE, cysH, cysK, cysN, cysQ, dps, eno, fda, gap, gap2, gdh, gnd, glyA, hom, hom^{FBR}, lysC, lysC^{FBR}, metA, metB, metE, metH, metY, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwa1, zwf and zwf A213T, which are compiled and explained in Table 3.

**Table 3**

| Genes and alleles of methionine production | | | |
|---|---|---|---|
| **Name** | **Name of the enzyme or protein coded for** | **Reference** | **Accession number** |
| accBC | acyl-CoA carboxylase EC 6.3.4.14 | Jäger *et al*. Archives of Microbiology (1996) 166:76-82 EP1108790; | U35023 |
| | | WO0100805 | AX123524 |
| | | | AX066441 |
| accDA | acetyl-CoA carboxylase | EP1055725 | |
| | EC 6.4.1.2 | EP1108790 | AX121013 |
| | | WO0100805 | AX066443 |
| aecD | cystathionine beta-lyase EC 4.4.1.8 | Rossol *et al.,* Journal of Bacteriology 174:2968-2977 (1992) | M89931 |
| cstA | carbon starvation protein A | EP1108790 | AX120811 |
| | | WO0100804 | AX066109 |
| cysD | sulfate adenylyltransferase subunit II EC 2.7.7.4 (sulfate adenylyltransferase small chain) | EP1108790 | AX123177 |
| cysE | serine acetyltransferase | EP1108790 | AX122902 |
| | EC 2.3.1.30 | WO0100843 | AX063961 |
| cysH | 3'-phosphoadenyl sulfate reductase | EP1108790 | AX123178 |
| | EC 1.8.99.4 (3'-phosphoadenosine 5'-phosphosulfate reductase) | WO0100842 | AX066001 |
| cysK | cysteine synthase | EP1108790 | AX122901 |
| | EC 4.2.99.8 | WO0100843 | AX063963 |
| cysN | sulfate adenylyltransferase | EP1108790 | AX123176 |
| | subunit I EC 2.7.7.4 (sulfate adenylyltransferase) | | AX127152 |
| cysQ | transport protein CysQ | EP1108790 | AX127145 |
| | (transporter cysQ) | WO0100805 | AX066423 |
| dps | DNA protection protein (protection during starvation protein) | EP1108790 | AX127153 |
| eno | enolase | EP1108790 | AX127146 |
| | EC 4.2.1.11 | WO0100844 | AX064945 |
| | | EP1090998 Hermann *et al*., Electrophoresis 19:3217-3221 (1998) | AX136862 |
| fda | fructose bisphosphate aldolase EC 4.1.2.13 | van der Osten et al., Molecular Microbiology 3:1625-1637 (1989) | X17313 |
| gap | glyceraldehyde-3-phosphate | EP1108790 | AX127148 |
| | dehydrogenase | WO0100844 | AX064941 |
| | EC 1.2.1.12 | Eikmanns *et al*., Journal of Bacteriology 174:6076-6086(1992) | X59403 |
| gap2 | glyceraldehyde-3-phosphate | EP1108790 | AX127146 |
| | dehydrogenase EC 1.2.1.12 (glyceraldehyde-3-phosphate dehydrogenase 2) | WO0100844 | AX064939 |
| gdh | glutamate dehydrogenase | EP1108790 | AX127150 |
| | EC 1.4.1.4 | WO0100844 | AX063811 |
| | | Boermann *et al.,* Molecular Microbiology | X59404 |
| | | 6:317-326 (1992) | X72855 |
| glyA | glycine/serine | EP1108790 | AX127146 |
| | hydroxymethyltransferase EC 2.1.2.1 | | AX121194 |
| gnd | 6-phosphogluconate dehydrogenase | EP1108790 | AX127147 |
| | EC 1.1.1.44 | | AX121689 |
| | | WO0100844 | AX065125 |
| hom | homoserine dehydrogenase EC 1.1.1.3 | Peoples *et al.,* Molecular Microbiology 2:63-72 (1988) | Y00546 |
| hom^{FBR} | homoserine dehydrogenase feedback resistant (fbr) EC 1.1.1.3 (homoserine dehydrogenase fbr) | Reinscheid et al., Journal of Bacteriology 173:3228-30 (1991) | |
| lysC | aspartate kinase | EP1108790 | AX120365 |
| | EC 2.7.2.4 | WO0100844 | AX063743 |
| | | Kalinowski et al., Molecular Microbiology 5:1197-209 (1991) | X57226 |
| lysC^{FBR} | aspartate kinase feedback resistant (fbr) EC 2.7.2.4 (aspartate kinase fbr) | see Table 2 | |
| metA | homoserine acetyltransferase EC 2.3.1.31 | Park *et al.,* Molecular Cells 8:286-94 (1998) | AF052652 |
| metB | cystathionine γ-lyase EC 4.4.1.1 (cystathionine gamma-synthase) | Hwang *et al.,* Molecular Cells 9:300-308 (1999) | AF126953 |
| metE | homocysteine methyltransferase | EP1108790 | AX127146 |
| | EC 2.1.1.14 | | AX121345 |
| metH | homocysteine methyltransferase | EP1108790 | AX127148 |
| | (vitamin B12-dependent) EC 2.1.1.14 | | AX121747 |
| metY | acetylhomoserine sulfhydrolase | EP1108790 | AX120810 |
| | | | AX127145 |
| msiK | sugar importer (multiple sugar import protein) | EP1108790 | AX120892 |
| opcA | glucose-6-phosphate dehydrogenase (subunit of glucose-6-phosphate dehydrogenase) | WO0104325 | AX076272 |
| oxyR | transcription regulator | EP1108790 | AX122198 |
| | (transcriptional regulator) | | AX127149 |
| ppc^{FBR} | phosphoenolpyruvate carboxylase feedback resistant EC 4.1.1.31 | EP0723011 WO0100852 | |
| ppc | phosphoenolpyruvate carboxylase | EP1108790 | AX127148 |
| | EC 4.1.1.31 | | AX123554 |
| | | O'Reagan *et al.,* Gene 77 (2) :237-251 (1989) | M25819 |
| pgk | phosphoglycerate kinase | EP1108790 | AX121838 |
| | EC 2.7.2.3 | | AX127148 |
| | | WO0100844 | AX064943 |
| | | Eikmanns, Journal of Bacteriology 174:6076-6086 (1992) | X59403 |
| pknA | protein kinase A | EP1108790 | AX120131 |
| | | | AX120085 |
| pknB | protein kinase B | EP1108790 | AX120130 |
| | | | AX120085 |
| pknD | protein kinase D | EP1108790 | AX127150 |
| | | | AX122469 |
| | | | AX122468 |
| pknG | protein kinase G | EP1108790 | AX127152 |
| | | | AX123109 |
| ppsA | phosphoenolpyruvate synthase | EP1108790 | AX127144 |
| | EC 2.7.9.2 | | AX120700 |
| | | | AX122469 |
| ptsH | phosphotransferase system protein H | EP1108790 | AX122210 |
| | EC 2.7.1.69 | | AX127149 |
| | (phosphotransferase system component H) | WO0100844 | AX069154 |
| ptsI | phosphotransferase system enzyme I | EP1108790 | AX122206 |
| | EC 2.7.3.9 | | AX127149 |
| ptsM | glucose-specific phosphotransferase system enzyme II EC 2.7.1.69 (glucose-phosphotransferase-system enzyme II) | Lee *et al*., FEMS Microbiology Letters 119(1-2):137-145 (1994) | L18874 |
| pyc | pyrvate carboxylase | WO9918228 | A97276 |
| | EC 6.4.1.1 | Peters-Wendisch *et al.,* Microbiology 144:915-927 (1998) | Y09548 |
| pyc P458S | pyrvate carboxylase EC 6.4.1.1 amino acid substitution P458S | EP1108790 | |
| sigC | sigma factor C | EP1108790 | AX120368 |
| | EC 2.7.7.6 (extracytoplasmic function alternative sigma factor C) | | AX120085 |
| sigD | RNA polymerase sigma factor D | EP1108790 | AX120753 |
| | EC 2.7.7.6 (RNA polymerase sigma factor) | | AX127144 |
| sigE | sigma factor E | EP1108790 | AX127146 |
| | EC 2.7.7.6 (extracytoplasmic function alternative sigma factor E) | | AX121325 |
| sigH | sigma factor H | EP1108790 | AX127145 |
| | EC 2.7.7.6 (sigma factor SigH) | | AX120939 |
| sigM | sigma factor M | EP1108790 | AX123500 |
| | EC 2.7.7.6 (sigma factor SigM) | | AX127153 |
| tal | transaldolase EC 2.2.1.2 | WO0104325 | AX076272 |
| thyA | thymidylate synthase | EP1108790 | AX121026 |
| | EC 2.1.1.45 | | AX127145 |
| tkt | transketolase EC 2.2.1.1 | Ikeda *et al.,* NCBI | AB023377 |
| tpi | triose-phosphate isomerase EC 5.3.1.1 | Eikmanns, Journal of Bacteriology 174:6076-6086 (1992) | X59403 |
| zwa1 | cell growth factor 1 (growth factor 1) | EP1111062 | AX133781 |
| zwf | glucose-6-phosphate-1-dehydrogenase | EP1108790 | AX127148 |
| | EC 1.1.1.49 | | AX121827 |
| | | WO0104325 | AX076272 |
| zwf A213T | glucose-6-phosphate-1-dehydrogenase EC 1.1.1.49 amino acid substitution A213T | EP1108790 | |

It may further be advantageous for the production of L-methionine, in addition to attenuating the import of methionine from the surrounding medium, to be achieved, for example, by attenuation of one or more genes selected from the group yaeC, abc and yaeE, at the same time to attenuate, especially exclude or reduce the expression of, one or more genes selected from the group of the genes or alleles that are not essential for growth or for methionine production.

These include *inter alia* the following open reading frames, genes or alles: brnQ, ccpA1, ccpA2, citA, citB, citE, ddh, gluA, gluB, gluC, gluD, luxR, luxS, lysR1, lysR2, lysR3, menE, metD, metK, pck, pgi, poxB and zwa2, which are compiled and explained in Table 4.

**Table 4**

| Genes and alleles that are not essential for methionine production | | | |
|---|---|---|---|
| **Gene name** | **Name of the enzyme or protein coded for** | **Reference** | **Accession number** |
| brnQ | branched-chain amino | Tauch *et al*., Archives of Microbiology 169(4):303-12 (1998) | M89931 |
| | acid carrier protein | | AX066841 |
| | (branched-chain amino | | AX127150 |
| | acid transport system | WO0100805 | |
| | carrier protein) | EP1108790 | |
| ccpA1 | catabolite control | WO0100844 | AX065267 |
| | protein (catabolite control protein A1) | EP1108790 | AX127147 |
| ccpA2 | catabolite control | WO0100844 | AX065267 |
| | protein (catabolite control protein A2) | EP1108790 | AX121594 |
| citA | sensor kinase CitA | EP1108790 | AX120161 |
| citB | transcription regulator CitB | EP1108790 | AX120163 |
| citE | citrate lyase | W00100844 | AX065421 |
| | EC 4.1.3.6 | EP1108790 | AX127146 |
| ddh | diaminopimelate dehydrogenase EC 1.4.1.16 | Ishino *et al*., Nucleic Acids Research 15: 3917 (1987) EP1108790 | S07384 AX127152 |
| gluA | glutamate transport ATP-binding protein | Kronemeyer *et al.,* Journal of Bacteriology 177(5):1152-8 (1995) | X81191 |
| gluB | glutamate binding protein | Kronemeyer *et al.,* Journal of Bacteriology 177(5):1152-8 (1995) | X81191 |
| gluC | glutamate transport permease (glutamate transport system permease) | Kronemeyer *et al.,* Journal of Bacteriology 177(5):1152-8 (1995) | X81191 |
| gluD | glutamate transport permease (glutamate transport system permease) | Kronemeyer *et al.,* Journal of Bacteriology 177(5):1152-8 (1995) | X81191 |
| luxR | transcription regulator | WO0100842 | AX065953 |
| | LuxR | EP1108790 | AX123320 |
| luxS | histidine kinase LuxS | EP1108790 | AX123323 |
| | | | AX127153 |
| lysR1 | transcription regulator | EP1108790 | AX064673 |
| | LysR1 | | AX127144 |
| lysR2 | transcription activator LysR2 (transcription regulator LysR2) | EP1108790 | AX123312 |
| lysR3 | transcription regulator | W00100842 | AX065957 |
| | LysR3 | EP1108790 | AX127150 |
| menE | O-succinylbenzoic acid | WO0100843 | AX064599 |
| | CoA ligase | EP1108790 | AX064193 |
| | EC 6.2.1.26 (O-succinylbenzoate-CoA ligase) | | AX127144 |
| metD | transcription regulator | EP1108790 | AX123327 |
| | MetD | | AX127153 |
| metK | methionine-adenosyl | WO0100843 | AX063959 |
| | transferase EC 2.5.1.6 (S-adenosylmethionine synthetase) | EP1108790 | AX127148 |
| pck | phosphoenolpyruvate | WO0100844 | AJ269506 |
| | carboxykinase | | AX065053 |
| pgi | glucose-6-phosphate | EP1087015 | AX136015 |
| | isomerase EC 5.3.1.9 | EP1108790 | AX127146 |
| poxB | pyruvate oxidase | WO0100844 | AX064959 |
| | EC 1.2.3.3 | EP1096013 | AX137665 |
| zwa2 | cell growth factor 2 | EP1106693 | AX113822 |
| | (growth factor 2) | EP1108790 | AX127146 |

Finally, it may be advantageous for the production of L-methionine, in addition to attenuating the gene arsR to exclude undesirable secondary reactions (Nakayama: "Breeding of Amino Acid Producing Microorganisms", in: Overproduction of Microbial Products, Krumphanzl, Sikyta, Vanek (eds.), Academic Press, London, UK, 1982).

The invention also provides the microorganisms produced according to the invention, which can be produced continuously or discontinuously by the batch process or by the fed batch or repeated fed batch process for the purposes of the production of L-amino acids. A summary of known cultivation methods is described in the textbook of Chmiel (Bioprozesstechnik 1. Einführung in die Bioverfahrenstechnik (Gustav Fischer Verlag, Stuttgart, 1991)) or in the textbook of Storhas (Bioreaktoren und periphere Einrichtungen (Vieweg Verlag, Braunschweig/Wiesbaden, 1994)).

The culture medium to be used must meet the requirements of the strains in question in a suitable manner. Descriptions of culture media for various microorganisms are to be found in the handbook "Manual of Methods for General Bacteriology" of the American Society for Bacteriology (Washington D.C., USA, 1981).

As the carbon source there may be used sugars and carbohydrates, such as, for example, glucose, sucrose, lactose, fructose, maltose, molasses, starch and cellulose, oils and fats, such as, for example, soybean oil, sunflower oil, groundnut oil and coconut oil, fatty acids, such as, for example, palmitic acid, stearic acid and linoleic acid, alcohols, such as, for example, glycerol and ethanol, and organic acids, such as, for example, acetic acid. These substances can be used individually or in the form of a mixture.

As the nitrogen source there may be used organic nitrogen-containing compounds, such as peptones, yeast extract, meat extract, malt extract, corn steep liquor, soybean flour and urea, or inorganic compounds, such as ammonium sulfate, ammonium chloride, ammonium phosphate, ammonium carbonate and ammonium nitrate. The nitrogen sources can be used individually or in the form of a mixture.

As the phosphorus source there may be used phosphoric acid, potassium dihydrogen phosphate or dipotassium hydrogen phosphate or the corresponding sodium-containing salts. The culture medium must also contain salts of metals, such as, for example, magnesium sulfate or iron sulfate, which are necessary for growth. Finally, essential growth substances, such as amino acids and vitamins, may be used in addition to the above-mentioned substances. Suitable precursors may also be added to the culture medium. The mentioned substances may be added to the culture in the form of a single batch, or they may be suitably fed in during the cultivation.

In order to control the pH value of the culture, basic compounds, such as sodium hydroxide, potassium hydroxide, ammonia or ammonia water, or acidic compounds, such as phosphoric acid or sulfuric acid, are expediently used. In order to control the development of foam, anti-foams, such as, for example, fatty acid polyglycol esters, may be used. In order to maintain the stability of plasmids, suitable substances having a selective action, such as, for example, antibiotics, may be added to the medium. In order to maintain aerobic conditions, oxygen or gas mixtures containing oxygen, such as, for example, air, are introduced into the culture. The temperature of the culture is normally from 20°C to 45°C and preferably from 25°C to 40°C. The culture is continued until the maximum amount of the desired product has formed. This aim is normally achieved within a period of from 10 hours to 160 hours.

With the methods of the invention it is possible to achieve an improvement of at least 0.5 %, at least 1 % or at least 2 % in the performance of the bacteria or of the fermentation process in respect of the product concentration (product per volume), the product yield (product formed per carbon source consumed), the product formation (product formed per volume and time) or other process parameters and combinations thereof.

Methods of determining L-amino acids are known from the prior art. The analysis may be carried out as described in Spackman et al. (Analytical Chemistry, 30, (1958), 1190) by anion-exchange chromatography with subsequent ninhydrin derivatisation, or it may be carried out by reversed phase HPLC, as described in Lindroth et al. (Analytical Chemistry (1979) 51: 1167-1174). The person skilled in the art will also find relevant information thereon in Ashman et al. (in: Tschesche (ed.), Modern Methods in Protein Chemistry. 155-172, de Gruyter, Berlin 1985).

The process according to the invention is used for the production of L-methionine by fermentation.

The concentration of L-methionine in the end product can optionally be adjusted to the desired value by the addition of L-methionine.

The present invention is explained hereinbelow by means of examples.

### Example 1 (not belonging to the invention)

### Preparation of an integration vector for integration mutagenesis of the malI gene

Chromosomal DNA is isolated from strain ATCC 13032 by the method of Eikmanns et al. (Microbiology 140: 1817-1828 (1994)).

On the basis of the known sequence of the malI gene for C. glutamicum, the following oligonucleotides are selected for the polymerase chain reaction:
malI-int1:
5' TCG AAT CCC TTC TTC TTG G 3'
malI-int2:
5' TTA GGA CCG GCG ATA TAG G 3'

The primers shown are synthesised by MWG Biotech (Ebersberg, Germany), and the PCR reaction is carried out according to the standared PCR method of Innis et al. (PCR protocols. A guide to methods and applications, 1990, Academic Press) using Taq polymerase from Boehringer Mannheim (Germany, product description Taq DNA polymerase, Product No. 1 146 165). With the aid of the polymerase chain reaction, the primers permit the amplification of an internal fragment of the malI gene having a size of 338 bp. The product so amplified is investigated by electrophoresis in a 0.8 % agarose gel.

The amplified DNA fragment is ligated into vector pCR2.1-TOPO (Mead et al. (1991), Bio/Technology 9:657-663) using the TOPO TA Cloning Kit from Invitrogen Corporation (Carlsbad, CA, USA; Catalog Number K4500-01).

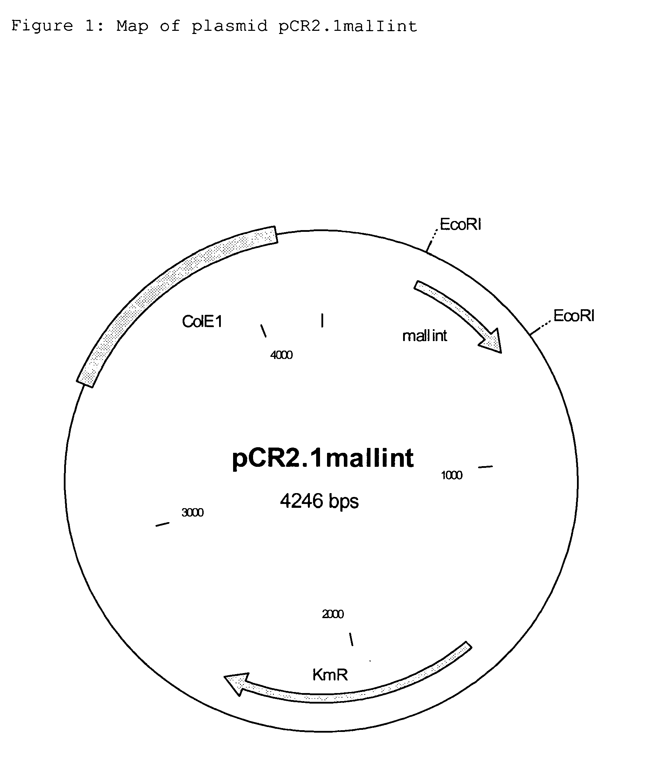

E. coli strain TOP10 is then electroporated with the ligation batch (Hanahan, in: DNA Cloning. A Practical Approach, Vol. I, IRL-Press, Oxford, Washington DC, USA, 1985). The selection of plasmid-carrying cells is carried out by plating out the transformation batch on LB agar (Sambrook et al., Molecular Cloning: a Laboratory Manual, 2nd Ed., Cold Spring Harbor Laboratory Press, Cold Spring Harbor, N.Y., 1989) which has been supplemented with 50 mg/l kanamycin. Plasmid DNA is isolated from a transformant with the aid of the QIAprep Spin Miniprep Kit from Qiagen and is tested by restriction with the restriction enzyme EcoRI and subsequent agarose gel electrophoresis (0.8 %). The plasmid is named pCR2.1malIint and is shown in Figure 1.

### Example 2 (not belonging to the invention)

### Integration mutagenesis of the malI gene in the strain DSM 16835

Vector pCR2.1malIint mentioned in Example 1 is electroporated in Corynebacterium glutamicum DSM 16835 by the electroporation method of Tauch et al. (FEMS Microbiological Letters, 123:343-347 (1994)). Strain DSM 16835 is an AEC-resistant lysine producer, the strain is described in Menkel et al. (Applied and Environmental Microbiology 55 (3): 684-688 (1989) under the name MH20-22B. Vector pCR2.lmalIint is unable to replicate independently in DSM 16835 and is retained in the cell only if it has integrated into the chromosome of DSM 16835. The selection of clones with pCR2.1malIint integrated into the chromosome is effected by plating out the electroporation batch on LB agar (Sambrook et al., Molecular Cloning: A Laboratory Manual, 2nd Ed., Cold Spring Harbor Laboratory Press, Cold Spring Harbor, N.Y.) which has been supplemented with 15 mg/l kanamycin.

A selected kanamycin-resistant clone which has the plasmid pCR2.1malIint inserted within the chromosomal malI gene of DSM 16835 was designated DSM16835::pCR2.1malIint.

### Example 3 (not belonging to the invention)

### Production of lysine

The C. glutamicum strain DSM16835::pCR2.1malIint obtained in Example 2 is cultivated in a nutrient medium suitable for the production of lysine, and the lysine content in the culture supernatant is determined.

To that end, the strain is first incubated for 24 hours at 33°C on agar plate with the appropriate antibiotic (brain-heart agar with kanamycin (25 mg/l) Starting from this agar plate culture, a pre-culture is inoculated (10 ml of medium in a 100 ml Erlenmeyer flask). CgIII complete medium is used as the medium for the pre-culture.

| | Cg III medium |
|---|---|
| NaCl | 2.5 g/l |
| Bacto-peptone | 10 g/l |
| Bacto-yeast extract | 10 g/l |
| Glucose (autoclaved separately) | 2% (w/v) |
| The pH value is adjusted to pH 7.4 | |

Kanamycin (25 mg/l) is added thereto. The pre-culture is incubated for 16 hours on a shaker at 33°C and 240 rpm. A main culture is inoculated from this pre-culture, so that the initial OD (660 nm) of the main culture is 0.1 OD. MM medium is used for the main culture.

| | MM medium |
|---|---|
| CSL (corn steep liquor) | 5 g/l |
| MOPS (morpholinopropanesulfonic acid) | 20 g/l |
| Glucose (autoclaved separately) | 50 g/l |

| Salts: | |
|---|---|
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0 mg/l |
| Biotin (sterilised by filtration) | 0.3 mg/l |
| Thiamin * HCl (sterilised by filtration) | 0.2 mg/l |
| Leucine (sterilised by filtration) | 0.1 g/l |
| CaCO₃ | 25 g/l |

CSL, MOPS and the salt solution are adjusted to pH 7 with ammonia water and autoclaved. The sterile substrate and vitamin solutions are then added, as well as the dry autoclaved CaCO₃.

Cultivation is carried out in a volume of 10 ml in a 100 ml Erlenmeyer flask with baffles. Kanamycin (25 mg/l) is added. Cultivation is carried out at 33°C and 80 % humidity.

After 72 hours, the OD is determined at a measuring wavelength of 660 nm using a Biomek 1000 (Beckmann Instruments GmbH, Munich). The amount of lysine that has formed is determined using an amino acid analyser from Eppendorf-BioTronik (Hamburg, Germany) by ion-exchange chromatography and post-column derivatisation with ninhydrin detection.

The result of the test is shown in Table 1.

**Table 1**

| Strain | OD | Lysine HCl |
|---|---|---|
| | (660 nm) | g/l |
| DSM16835 | 7.4 | 12.84 |
| DSM16835::pCR2.1malIint | 7.7 | 14.1 |

### Example 4 (not belonging to the invention)

### Preparation of an integration vector for integration mutagenesis of the gatR gene

Chromosomal DNA is isolated from strain ATCC 13032 by the method of Eikmanns et al. (Microbiology 140: 1817-1828 (1994)).

On the basis of the known sequence of the gatR gene for C. glutamicum, the following oligonucleotides are selected for the polymerase chain reaction:
gatR-int1:
5' AAG AGG CGT TGT GTA CTG C 3'
gatR-int2:
5' TGG TCA AAC TCC TCA ATC G 3'

The primers shown are synthesised by MWG Biotech (Ebersberg, Germany), and the PCR reaction is carried out according to the standared PCR method of Innis et al. (PCR Protocols. A Guide to Methods and Applications, 1990, Academic Press) using Taq polymerase from Boehringer Mannheim (Germany, product description Taq DNA polymerase, Product No. 1 146 165). With the aid of the polymerase chain reaction, the primers permit the amplification of an internal fragment of the gatR gene having a size of 466 bp.

The product so amplified is investigated by electrophoresis in a 0.8 % agarose gel.

The amplified DNA fragment is ligated into vector pCR2.1-TOPO (Mead et al. (1991), Bio/Technology 9:657-663) using the TOPO TA Cloning Kit from Invitrogen Corporation (Carlsbad, CA, USA; Catalog Number K4500-01).

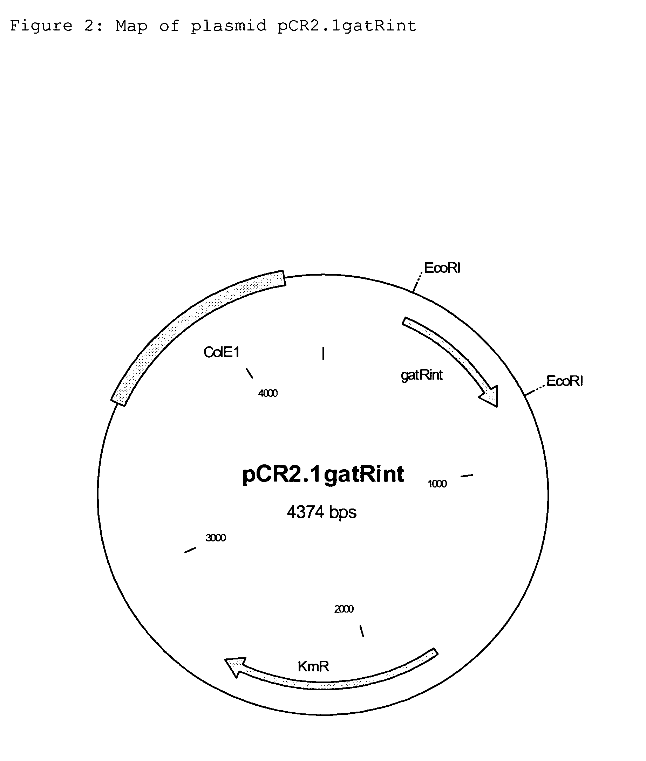

E. coli strain TOP10 is then electroporated with the ligation batch (Hanahan, in: DNA Cloning. A Practical Approach, Vol. I, IRL-Press, Oxford, Washington DC, USA, 1985). The selection of plasmid-carrying cells is carried out by plating out the transformation batch on LB agar (Sambrook et al., Molecular Cloning: A Laboratory Manual, 2nd Ed., Cold Spring Harbor Laboratory Press, Cold Spring Harbor, N.Y., 1989) which has been supplemented with 50 mg/l kanamycin. Plasmid DNA is isolated from a transformant with the aid of the QIAprep Spin Miniprep Kit from Qiagen and is tested by restriction with the restriction enzyme EcoRI and subsequent agarose gel electrophoresis (0.8 %). The plasmid is named pCR2.1gatRint and is shown in Figure 2.

### Example 5 (not belonging to the invention)

### Integration mutagenesis of the gatR gene in strain DSM 16835

Vector pCR2.1gatRint mentioned in Example 4 is electroporated in Corynebacterium glutamicum DSM 16835 by the electroporation method of Tauch et al. (FEMS Microbiological Letters, 123:343-347 (1994)). Strain DSM 16835 is an AEC-resistant lysine producer, the strain is described in Menkel et al. (Applied and Environmental Microbiology 55 (3): 684-688 (1989) under the name MH20-22B. Vector pCR2.1gatRint is unable to replicate independently in DSM 16835 and is retained in the cell only if it has integrated into the chromosome of DSM 16835. The selection of clones with pCR2.1gatRint integrated into the chromosome is effected by plating out the electroporation batch on LB agar (Sambrook et al., Molecular Cloning: A Laboratory Manual. 2nd Ed., Cold Spring Harbor Laboratory Press, Cold Spring Harbor, N.Y.) which has been supplemented with 15 mg/l kanamycin.

A selected kanamycin-resistant clone which has the plasmid pCR2.1gatRint mentioned in Example 4 inserted within the chromosomal gatR gene of DSM 16835 was designated DSM16835::pCR2.1gatRint.

### Example 6 (not belonging to the invention)

### Production of lysine

The C. glutamicum strain DSM16835::pCR2.1gatRint obtained in Example 5 is cultivated in a nutrient medium suitable for the production of lysine, and the lysine content in the culture supernatant is determined.

To that end, the strain is first incubated for 24 hours at 33°C on agar plate with the appropriate antibiotic (brain-heart agar with kanamycin (25 mg/l). Starting from this agar plate culture, a pre-culture is inoculated (10 ml of medium in a 100 ml Erlenmeyer flask). CgIII complete medium is used as the medium for the pre-culture.

| | Cg III medium |
|---|---|
| NaCl | 2.5 g/l |
| Bacto-peptone | 10 g/l |
| Bacto-yeast extract | 10 g/l |
| Glucose (autoclaved separately) | 2% (w/v) |
| The pH value is adjusted to pH 7.4 | |

Kanamycin (25 mg/l) is added thereto. The pre-culture is incubated for 16 hours on a shaker at 33°C and 240 rpm. A main culture is inoculated from this pre-culture, so that the initial OD (660 nm) of the main culture is 0.1 OD. MM medium is used for the main culture.

| | MM medium |
|---|---|
| CSL (corn steep liquor) | 5 g/l |
| MOPS (morpholinopropanesulfonic acid) | 20 g/l |
| Glucose (autoclaved separately) | 50 g/l |

| Salts: | |
|---|---|
| (NH₄) ₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0 mg/l |
| Biotin (sterilised by filtration) | 0.3 mg/l |
| Thiamin * HCl (sterilised by filtration) | 0.2 mg/l |
| Leucine (sterilised by filtration) | 0.1 g/l |
| CaCO₃ | 25 g/l |

CSL, MOPS and the salt solution are adjusted to pH 7 with ammonia water and autoclaved. The sterile substrate and vitamin solutions are then added, as well as the dry autoclaved CaCO₃.

Cultivation is carried out in a volume of 10 ml in a 100 ml Erlenmeyer flask with baffles. Kanamycin (25 mg/l) is added. Cultivation is carried out at 33°C and 80 % humidity.

After 72 hours, the OD is determined at a measuring wavelength of 660 nm using a Biomek 1000 (Beckmann Instruments GmbH, Munich). The amount of lysine that has formed is determined using an amino acid analyser from Eppendorf-BioTronik (Hamburg, Germany) by ion-exchange chromatography and post-column derivatisation with ninhydrin detection.

The result of the test is shown in Table 2.

**Table 2**

| Strain | OD | Lysine HCl |
|---|---|---|
| | (660 nm) | g/l |
| DSM16835 | 7.6 | 13.57 |
| DSM16835::pCR2.1gatRint | 7.8 | 14.92 |

### Example 7 (not belonging to the invention)

### Preparation of an integration vector for integration mutagenesis of the glpR2 gene

Chromosomal DNA is isolated from strain ATCC 13032 by the method of Eikmanns et al. (Microbiology 140: 1817-1828 (1994)).

On the basis of the known sequence of the glpR2 gene for C. glutamicum, the following oligonucleotides are selected for the polymerase chain reaction:
glpR2-int1:
5' AAC CTG CCT TGG TTA AAG G 3'
glpR2-int2:
5' ATC TAG CGT GTG GTC ATC G 3'

The primers shown are synthesised by MWG Biotech (Ebersberg, Germany), and the PCR reaction is carried out according to the standared PCR method of Innis et al. (PCR Protocols. A Guide to Methods and Applications, 1990, Academic Press) using Taq polymerase from Boehringer Mannheim (Germany, product description Taq DNA polymerase, Product No. 1 146 165). With the aid of the polymerase chain reaction, the primers permit the amplification of an internal fragment of the glpR2 gene having a size of 539 bp. The product so amplified is investigated by electrophoresis in a 0.8 % agarose gel.

The amplified DNA fragment is ligated into vector pCR2.1-TOPO (Mead et al. (1991), Bio/Technology 9:657-663) using the TOPO TA Cloning Kit from Invitrogen Corporation (Carlsbad, CA, USA; Catalog Number K4500-01).

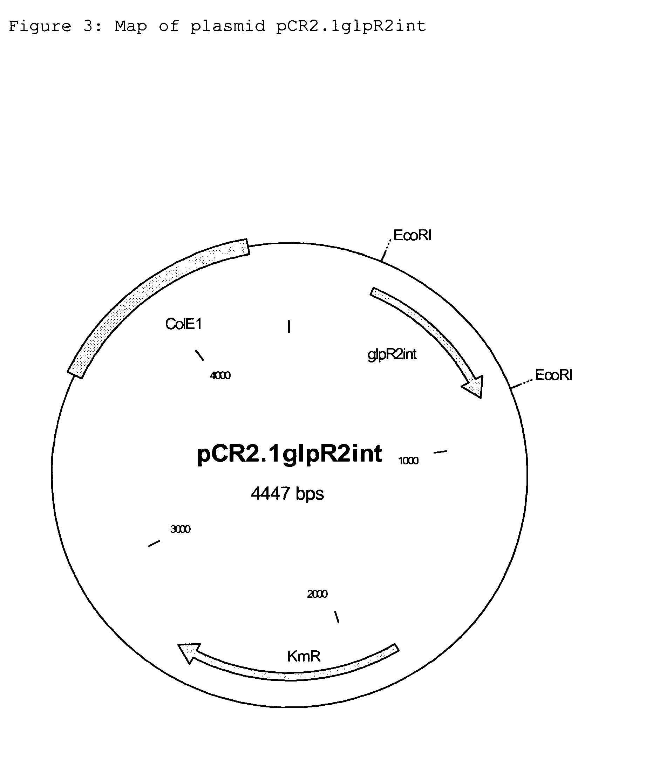

E. coli strain TOP10 is then electroporated with the ligation batch (Hanahan, in: DNA Cloning. A Practical Approach, Vol. I, IRL-Press, Oxford, Washington DC, USA, 1985). The selection of plasmid-carrying cells is carried out by plating out the transformation batch on LB agar (Sambrook et al., Molecular Cloning: A Laboratory Manual, 2nd Ed., Cold Spring Harbor Laboratory Press, Cold Spring Harbor, N.Y., 1989) which has been supplemented with 50 mg/l kanamycin. Plasmid DNA is isolated from a transformant with the aid of the QIAprep Spin Miniprep Kit from Qiagen and is tested by restriction with the restriction enzyme EcoRI and subsequent agarose gel electrophoresis (0.8 %). The plasmid is named pCR2.1g1pR2int and is shown in Figure 3.

### Example 8 (not belonging to the invention)

### Integration mutagenesis of the glpR2 gene in strain DSM 16835

Vector pCR2.1g1pR2int mentioned in Example 7 is electroporated in Corynebacterium glutamicum DSM 16835 by the electroporation method of Tauch et al. (FEMS Microbiological Letters, 123:343-347 (1994)). Strain DSM 16835 is an AEC-resistant lysine producer, the strain is described in Menkel et al. (Applied and Environmental Microbiology 55 (3): 684-688 (1989) under the name MH20-22B. Vector pCR2.1glpR2int is unable to replicate independently in DSM 16835 and is retained in the cell only if it has integrated into the chromosome of DSM 16835. The selection of clones with pCR2.1glpR2int integrated into the chromosome is effected by plating out the electroporation batch on LB agar (Sambrook et al., Molecular Cloning: A Laboratory Manual. 2nd Ed., Cold Spring Harbor Laboratory Press, Cold Spring Harbor, N.Y.) which has been supplemented with 15 mg/l kanamycin.

A selected kanamycin-resistant clone which has the plasmid pCR2.1glpR2int mentioned in Example 7 inserted within the chromosomal glpR2 gene of DSM 16835 was designated DSM16835::pCR2.1g1pR2int.

### Example 9 (not belonging to the invention)

### Production of lysine

The C. glutamicum strain DSM16835::pCR2.1glpR2int obtained in Example 8 is cultivated in a nutrient medium suitable for the production of lysine, and the lysine content in the culture supernatant is determined.

To that end, the strain is first incubated for 24 hours at 33°C on agar plate with the appropriate antibiotic (brain-heart agar with kanamycin (25 mg/l). Starting from this agar plate culture, a pre-culture is inoculated (10 ml of medium in a 100 ml Erlenmeyer flask). CgIII complete medium is used as the medium for the pre-culture.

| | Cg III medium |
|---|---|
| NaCl | 2.5 g/l |
| Bacto-peptone | 10 g/l |
| Bacto-yeast extract | 10 g/l |
| Glucose (autoclaved separately) | 2% (w/v) |
| The pH value is adjusted to pH 7.4 | |

Kanamycin (25 mg/l) is added thereto. The pre-culture is incubated for 16 hours on a shaker at 33°C and 240 rpm. A main culture is inoculated from this pre-culture, so that the initial OD (660 nm) of the main culture is 0.1 OD. MM medium is used for the main culture.

| | MM medium |
|---|---|
| CSL (corn steep liquor) | 5 g/l |
| MOPS (morpholinopropanesulfonic acid) | 20 g/l |
| Glucose (autoclaved separately) | 50 g/l |

| Salts: | |
|---|---|
| (NH₄)₂SO₄ | 25 g/l |
| KH₂PO₄) | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0 mg/l |
| Biotin (sterilised by filtration) | 0.3 mg/l |
| Thiamin * HCl (sterilised by filtration) | 0.2 mg/l |
| Leucine (sterilised by filtration) | 0.1 g/l |
| CaCO₃ | 25 g/l |

CSL, MOPS and the salt solution are adjusted to pH 7 with ammonia water and autoclaved. The sterile substrate and vitamin solutions are then added, as well as the dry autoclaved CaCO₃.

Cultivation is carried out in a volume of 10 ml in a 100 ml Erlenmeyer flask with baffles. Kanamycin (25 mg/l) is added. Cultivation is carried out at 33°C and 80 % humidity.

After 72 hours, the OD is determined at a measuring wavelength of 660 nm using a Biomek 1000 (Beckmann Instruments GmbH, Munich). The amount of lysine that has formed is determined using an amino acid analyser from Eppendorf-BioTronik (Hamburg, Germany) by ion-exchange chromatography and post-column derivatisation with ninhydrin detection.

The result of the test is shown in Table 3.

**Table 3**

| Strain | OD | Lysine HCl |
|---|---|---|
| | (660 nm) | g/l |
| DSM16835 | 7.6 | 13.57 |
| DSM16835::pCR2.1g1pR2int | 8.4 | 13.97 |

### Brief description of the Figures:

Figure 1: Map of plasmid pCR2.1malIin
Figure 2: Map of plasmid pCR2.1gatRint
Figure 3: Map of plasmid pCR2.1glpR2int

When indicating the number of base pairs, the values are approximate values obtained within the scope of the reproducibility of measurements.

The abbreviations and names used have the following meanings:

| | |
|---|---|
| KmR: | kanamycin resistance gene |
| EcoRI: | cleavage site of the restriction enzyme EcoRI |
| malIint: | internal fragment of the malI gene |
| gatRint: | internal fragment of the gatR gene |
| glpR2int: | internal fragment of the glpR2 gene |
| ColE1: | origin of replication of plasmid ColE1 |

## Claims

1. Process for the production of L-methionine by fermentation of coryneform bacteria, **characterised in that** there are used bacteria producing L-methionine in which the arsR gene having the sequence of 
ATGACCTCCGTTATCCCATCCAGCGCAGACAGTGCCACAGAGCAGCGCTTCGCGCCGCTAAAATCCGTAC
CCAGCAGGATCACGGACATCGCCGAAGAGGCCAGCGATTTCTACAAGGCACTCGGTGACACCACCCGCCT
AGAAATCTTGTACCTGATTTTCACTACAAAAAGCTCGCGGGTGAGCGCGAATGCCCTGGCACACACACTC
AATATTTCCGCCCCCACGGTGACCCACCATATGAAAAAGCTCATCGCAGCTAATTTGGTCAACCGTGAAC
AATGCGGAAAGTGGGCGTATTTCAGCATTAATTCCGCTCATATTGATACGGTTGCTGAAATTTTCGCCCA
CGCGTCC
 which codes for the transcription regulator ArsR has been attenuated.

2. Process according to claim 1 in which said gene has been excluded or expressed at a low lewel.

3. Process according to claim 1 or 2, **characterised in that** the following steps are carried out:
a) fermentation of said coryneform bacteria;
b) concentration of the product in the medium or in the cells of the bacteria, and
c) isolation of the desired L-methionine, portions (> 0 to 100 %) or the totality of constituents of the fermentation liquor and/or of the biomass optionally remaining in the end product.

4. Process according to claim 1 to 3, **characterised in that** there are used bacteria in which further genes of the biosynthesis pathway of the desired L-methionine are additionally enhanced.

5. Process according to claim 1 to 4, **characterised in that** there are used bacteria in which the metabolic pathways that reduce the formation of the desired L-methionine are at least partially excluded.

6. Process according to claim 1 to 5, **characterised in that** the regulatory and/or catalytic properties of the polypeptide (enzyme protein) coded for by the sequence of the arsR gene according to claim 1 are reduced.

7. Process according to claim 1 to 6, **characterised in that**, for the production of L-methionine there are fermented coryneform microorganisms in which one or more genes selected from the group
7.1 the gene lysC coding for a feedback-resistant aspartate kinase,
7.2 the gene gap coding for glyceraldehyde-3-phosphate dehydrogenase,
7.3 the gene pyc coding for pyruvate carboxylase,
7.4 the gene zwf coding for glucose-6-phosphate dehydrogenase,
7.5 the gene mqo coding for malate:quinone oxidoreductase,
7.6 the gene zwa1 coding for the Zwa1 protein,
7.7 the gene tpi coding for triose-phosphate isomerase, and
7.8 the gene pgk coding for 3-phosphoglycerate kinase is/are at the same time enhanced.

8. Process according to claim 7, **characterized in that** said gene(s) is/are overexpressed.

9. Process according to claim 1 to 6, **characterised in that**, for the production of L-methionine there are fermented coryneform microorganisms in which one or more genes selected from the group
9.1 the gene ccpA1 coding for a catabolite control protein A,
9.2 the pck gene coding for phosphoenol pyruvate carboxykinase,
9.3 the pgi gene coding for glucose-6-phosphate isomerase,
9.4 the gene poxB coding for pyruvate oxidase,
9.5 the gene fda coding for fructose bisphosphate aldolase,
9.6 the gene zwa2 coding for the Zwa2 protein,
9.7 the gene thrB coding for homoserine kinase,
9.8 the gene ilvA coding for threonine dehydratase and
9.9 the gene thrC coding for threonine synthase, is/are at the same time attenuated.

10. Process according to claim 9, **characterized in that** said gene(s) is/are excluded or the expression thereof is/are reduced.

11. Process according to one or more of claims 1 to 10, **characterised in that** microorganisms of the species Corynebacterium glutamicum are used.

12. Coryneform bacteria in which the arsR gene having the sequence according to claim 1 is present in attenuated form.

13. Coryneform bacteria according to claim 12, wherein said gene is excluded or expressed at a low level.

## Patentansprüche

1. Verfahren zur Produktion von L-Methionin durch Fermentation coryneformer Bakterien, **dadurch gekennzeichnet, dass** dabei L-Methionin produzierende Bakterien verwendet werden, in denen das arsR-Gen mit der Sequenz, 
ATGACCTCCGTTATCCCATCCAGCGCAGACAGTGCCACAGAGCAGCGCTTCGCGCCGCTAAAATCCGTAC
CCAGCAGGATCACGGACATCGCCGAAGAGGCCAGCGATTTCTACAAGGCACTCGGTGACACCACCCGCCT
AGAAATCTTGTACCTGATTTTCACTACAAAAAGCTCGCGGGTGAGCGCGAATGCCCTGGCACACACACTC
AATATTTCCGCCCCCACGGTGACCCACCATATGAAAAAGCTCATCGCAGCTAATTTGGTCAACCGTGAAC
AATGCGGAAAGTGGGCGTATTTCAGCATTAATTCCGCTCATATTGATACGGTTGCTGAAATTTTCGCCCA
CGCGTCC
 das für den Transkriptionsregulator ArsR codiert, abgeschwächt, wurde.

2. Verfahren nach Anspruch 1, bei dem das Gen ausgeschlossen oder auf einem niedrigen Niveau exprimiert wurde.

3. Verfahren nach Anspruch 1 oder 2, **dadurch gekennzeichnet, dass** die folgenden Schritte durchgeführt werden:
a) Fermentation der coryneformen Bakterien;
b) Anreicherung des Produkts im Medium oder in den Bakterienzellen und
c) Isolierung des gewünschten L-Methionins sowie teilweise (> 0 bis 100%) oder vollständige Isolierung der Bestandteile der Fermentationsbrühe und/oder der gegebenenfalls im Endprodukt verbleibenden Biomasse.

4. Verfahren nach Anspruch 1 bis 3, **dadurch gekennzeichnet, dass** dabei Bakterien verwendet werden, in denen weitere Gene des Biosynthesewegs des gewünschten L-Methionins zusätzlich verstärkt sind.

5. Verfahren nach Anspruch 1 bis 4, **dadurch gekennzeichnet, dass** dabei Bakterien verwendet werden, in denen die Stoffwechselwege, die die Bildung des gewünschten L-Methionins vermindern, wenigstens teilweise ausgeschlossen sind.

6. Verfahren nach Anspruch 1 bis 5, **dadurch gekennzeichnet, dass** die regulatorischen und/oder katalytischen Eigenschaften des Polypeptids (Enzymprotein), das von der Sequenz des arsR-Gens nach Anspruch 1 codiert wird, vermindert sind.

7. Verfahren nach Anspruch 1 bis 6, **dadurch gekennzeichnet, dass** dabei zur Produktion von L-Methionin coryneforme Mikroorganismen fermentiert werden, in denen ein Gen bzw. mehrere Gene, ausgewählt aus der Gruppe:
7.1 das für eine Feedback-resistente Aspartatkinase codierende Gen lysC,
7.2 das für Glyceraldehyd-3-phosphat-Dehydrogenase codierende Gen gap,
7.3 das für Pyruvat-Carboxylase codierende Gen pyc,
7.4 das für Glucose-6-phosphat-Dehydrogenase codierende Gen zwf,
7.5 das für Malat:Chinon-Oxidoreduktase codierende Gen mqo,
7.6 das für das Protein Zwa1 codierende Gen zwa1,
7.7 das für Triosephosphat-Isomerase codierende Gen tpi und
7.8 das für 3-Phosphoglycerat-Kinase codierende Gen pgk
gleichzeitig verstärkt ist bzw. sind.

8. Verfahren nach Anspruch 7, **dadurch gekennzeichnet, dass** das Gen bzw. die Gene überexprimiert ist bzw.sind.

9. Verfahren nach Anspruch 1 bis 6, **dadurch gekennzeichnet, dass** dabei zur Produktion von L-Methionin coryneforme Mikroorganismen fermentiert werden, in denen ein Gen bzw. mehrere Gene, ausgewählt aus der Gruppe:
9.1 das für ein Katabolit-Kontrollprotein A codierende Gen ccpA1,
9.2 das für Phosphoenolpyruvat-Carboxykinase codierende pck-Gen,
9.3 das für Glucose-6-phosphat-Isomerase codierende pgi-Gen,
9.4 das für Pyruvat-Oxidase codierende Gen poxB,
9.5 das für Fructose-bisphosphat-Aldolase codierende Gen fda,
9.6 das für das Protein Zwa2 codierende Gen zwa2, 9.7 das für Homoserin-Kinase codierende Gen thrB,
9.8 das für Threonin-Dehydratase codierende Gen ilvA und
9.9 das für Threonin-Synthase codierende Gen thrC,
gleichzeitig abgeschwächt, ist bzw. sind.

10. Verfahren nach Anspruch 9, **dadurch gekennzeichnet, dass** das Gen bzw. die Gene ausgeschlossen ist bzw. sind oder dessen bzw. deren Expression reduziert ist.

11. Verfahren nach einem oder mehreren der Ansprüche 1 bis 10, **dadurch gekennzeichnet, dass** Mikroorganismen der Spezies Corynebacterium glutamicum verwendet werden.

12. Coryneforme Bakterien, in denen das arsR-Gen mit der Sequenz nach Anspruch 1 in abgeschwächter Form vorliegt.

13. Coryneforme Bakterien nach Anspruch 12, wobei das Gen ausgeschlossen ist oder auf einem niedrigen Niveau exprimiert wird.

## Revendications

1. Procédé pour la production de L-méthionine par fermentation de bactéries corynéformes, **caractérisé en ce qu'**on utilise des bactéries produisant de la L-méthionine dans lesquelles le gène arsR ayant la séquence 
ATGACCTCCGTTATCCCATCCAGCGCAGACAGTGCCACAGAGCAGCGCTTCGCGCCGCTAAAATCCGTAC
CCAGCAGGATCACGGACATCGCCGAAGAGGCCAGCGATTTCTACAAGGCACTCGGTGACACCACCCGCCT
AGAAATCTTGTACCTGATTTTCACTACAAAAAGCTCGCGGGTGAGCGCGAATGCCCTGGCACACACACTC
AATATTTCCGCCCCCACGGTGACCCACCATATGAAAAAGCTCATCGCAGCTAATTTGGTCAACCGTGAAC
AATGCGGAAAGTGGGCGTATTTCAGCATTAATTCCGCTCATATTGATACGGTTGCTGAAATTTTCGCCCA
CGCGTCC
 qui code pour le régulateur de transcription ArsR a été atténué.

2. Procédé selon la revendication 1 dans lequel ledit gène a été exclu ou exprimé à un faible niveau.

3. Procédé selon la revendication 1 ou 2, **caractérisé en ce que** les étapes suivantes sont effectuées :
a) la fermentation desdites bactéries corynéformes ;
b) la concentration du produit dans le milieu ou dans les cellules des bactéries ; et
c) l'isolement de la L-méthionine souhaitée, de parties (> 0 à 100 %) ou de la totalité de constituants de la liqueur de fermentation et/ou de la biomasse restant facultativement dans le produit final.

4. Procédé selon les revendications 1 à 3, **caractérisé en ce qu'**on utilise des bactéries dans lesquelles d'autres gènes de la voie de biosynthèse de la L-méthionine souhaitée sont de plus amplifiés.

5. Procédé selon les revendications 1 à 4, **caractérisé en ce qu'**on utilise des bactéries dans lesquelles les voies métaboliques qui réduisent la formation de la L-méthionine souhaitée sont au moins en partie exclues.

6. Procédé selon les revendications 1 à 5, **caractérisé en ce que** les propriétés régulatrices et/ou catalytiques du polypeptide (de la protéine enzymatique) codé par la séquence du gène arsR selon la revendication 1 sont réduites.

7. Procédé selon les revendications 1 à 6, **caractérisé en ce que**, pour la production de L-méthionine il y a des microorganismes corynéformes fermentés dans lesquels un ou plusieurs gènes choisis parmi
7.1 le gène lysC codant pour une aspartate kinase résistante à la rétroaction,
7.2 le gène gap codant pour la glycéraldéhyde-3-phosphate déshydrogénase,
7.3 le gène pyc codant pour la pyruvate carboxylase,
7.4 le gène zwf codant pour la glucose-6-phosphate déshydrogénase,
7.5 le gène mqo codant pour la malate:quinone oxydoréductase,
7.6 le gène zwa1 codant pour la protéine Zwa1,
7.7 le gène tpi codant pour la triose-phosphate isomérase et
7.8 le gène pgk codant pour la 3-phosphoglycérate kinase
sont en même temps amplifiés.

8. Procédé selon la revendication 7, **caractérisé en ce que** ledit ou lesdits gènes sont surexprimés.

9. Procédé selon les revendications 1 à 6, **caractérisé en ce que**, pour la production de L-méthionine il y a des microorganismes corynéformes fermentés dans lesquels un ou plusieurs gènes choisis parmi
9.1 le gène ccpA1 codant pour un catabolite control protein A,
9.2 le gène pck codant pour la phosphoénolpyruvate carboxykinase,
9.3 le gène pgi codant pour la glucose-6-phosphate isomérase,
9.4 le gène poxB codant pour la pyruvate oxydase,
9.5 le gène fda codant pour la fructose-bisphosphate aldolase,
9.6 le gène zwa2 codant pour la protéine Zwa2,
9.7 le gène thrB codant pour l'homosérine kinase,
9.8 le gène ilvA codant pour la thréonine déshydratase et
9.9 le gène thrC codant pour la thréonine synthase, sont en même temps atténués.

10. Procédé selon la revendication 9, **caractérisé en ce que** ledit ou lesdits gènes sont exclus ou l'expression de ceux-ci est réduite.

11. Procédé selon une ou plusieurs des revendications 1 à 10, **caractérisé en ce que** des microorganismes de l'espèce *Corynebacterium glutamicum* sont utilisés.

12. Bactéries corynéformes dans lesquelles le gène arsR ayant la séquence selon la revendication 1 est présent sous forme atténuée.

13. Bactéries corynéformes selon la revendication 12, dans lesquelles ledit gène est exclu ou exprimé à un faible niveau.
